# EUROPEAN PATENT APPLICATION

(11) **EP 1 878 481 A1**
(43) Date of publication of application: **16.01.2008**
(21) Application number: 06116956.1
(22) Date of filing: 11.07.2006
(51) Int. Cl.: B01D 11/04, B01F 11/00

(54) **A method and a device for liquid-liquid extraction**

(71) Applicant: FOSS Analytical AB, 263 21 Höganäs (SE)
(72) Inventor: Wihlborg, Nils, 254 38 Helsingborg (SE)
(74) Representative: Grönlund, Tim Linus Wilhelm

(57) **Abstract**

A method for liquid-liquid extraction comprises: introducing a first liquid into a container (16) and introducing a second liquid into the container (16). The second liquid is substantially immiscible with the first liquid and a one of the first and second liquid contains a substance to be extracted and the other of the first and the second liquid exhibits a preferential affinity to the substance to be extracted. The method further comprises shaking the container (16), allowing the liquids to settle thereby establishing an interface between the liquids, and rotating the container to move the interface over substantially the entire inner surface of the container.

## Description

### Technical Field

The present invention relates to a method and a device for liquid-liquid extraction.

### Background of the Invention

In liquid-liquid extraction a substance that is dissolved in one liquid is transferred into another liquid. Liquid-liquid extraction may be used to separate a substance from a liquid in order to enrich or purify a liquid. Further, liquid-liquid extraction is used in order to extract bitter substances from beer such that the bitterness of the beer may be analysed.

The two liquids are mutually immiscible such that they may be easily separated. Further, the liquid, into which the substance is to be extracted, exhibits a preferential affinity towards the substance to urge the substance to be transferred between the liquids. The transfer of substance occurs in an interface between the two liquids. In order to accelerate extraction, the liquids are mixed so that the contact surface between the liquids is increased. When the extraction has been completed, the liquids are allowed to settle such that they will separate. The separation may be achieved by means of gravity, wherein the heavier liquid will settle at a bottom of a container. However, a problem with separation is that drops of one liquid may be trapped inside the other liquid. The separation may be improved and accelerated by centrifugating the liquids.

In many applications of liquid-liquid extraction, the extraction process is continuous. However, in extraction of bitter substances from beer, the extraction is performed in order to analyse the amount of bitter substances. This extraction is therefore performed on a beer sample to be analysed and may therefore be performed on each sample separately.

The European Brewery Committee publishes standardised analytical methods in "Analytica-EBC". In 2004 edition of "Analytica-EBC", Fachverlag Hans Carl of Nürnberg, Germany, a method of determining bitterness in beer is described in section 9.8. According to this method, the liquid-liquid extraction is performed in a centrifuge tube, which is shaken using a rotary shaker and thereafter centrifuged.

### Summary of the Invention

It is an object of the present invention to provide a method and a device for liquid-liquid extraction, wherein the separation of the two liquids is achieved in a controlled and effective manner.

This and other objects, which may become apparent from the following description, are achieved by a method according to claim 1 and a device according to claim 11. Specific embodiments of the invention are set out in the dependent claims.

Thus, according to one aspect of the invention, a method for liquid-liquid extraction comprises: introducing a first liquid into a container; and introducing a second liquid into the container. The second liquid is substantially immiscible with the first liquid and a one of the first and second liquid contains a substance to be extracted and the other of the first and the second liquid exhibits a preferential affinity to the substance to be extracted. The method further comprises shaking the container; allowing the liquids to settle thereby establishing an interface between the liquids, and rotating the container to move the interface over substantially the entire inner surface of the container.

By means of shaking the container, the first and second liquids are allowed to move vigorously inside the container and, therefore, the liquids will be thoroughly mixed.

When the extraction is completed, the first and second liquids will be separated after the shaking is stopped and the liquids have been allowed to settle. By means of the invention, the separation is improved by rotating the container. This implies that the first and second liquids may move along the walls of the container and, thus, any drops of a first liquid trapped inside the second liquid, or vice versa, may be captured and the separation of the liquids will be improved. The rotation of the container is especially effective, since it has been realized that drops of liquid trapped inside the other liquid often are situated along the walls of the container.

The shaking may be performed along a horizontal axis. Gravity will affect the first and second liquids to try to form layers along a horizontal axis of the container. A vigorous shaking in this direction will thus allow the liquids to be genuinely mixed in the entire container.

The term "horizontal axis" should be construed as an axis extending substantially along a direction perpendicular to a direction of gravity.

The container may be rotated around a horizontal axis. This implies that gravity will force the liquids to move along the walls of the container. Preferably, the container is rotated around a horizontal axis perpendicular to the axis along which shaking was performed.

The container may be rotated at least through an angle of 180°. Thus, the container is turned upside down from its original position and gravity will thus have forced the liquids to move along the entire walls of the container.

The rotation of the container may be performed gently in order to move the liquids along the walls of the container. This implies that the rotation merely brings trapped drops of liquid in contact with the separated phases to improve separation. The rotation is not vigorous to force the first and second liquids into separate parts of the container by means of the rotating movement.

The rotating may comprise rotating the container at least through an angle of 180° in a first direction and at least through an angle of 150° in a second, opposite direction. This rotation in the second, opposite direction implies that the separation of the liquids may be further improved.

Further, the rotating may comprise stopping the container in a position where the container is arranged in an upright position and a port of the container is facing upwards. This facilitates output of the second liquid from the container.

The method may further comprise introducing a third liquid into the container, which third liquid acts to break connections between the substance and the first liquid. The third liquid may be required in order to achieve the extraction.

The introducing of liquids may be controlled by increasing a space in the container in order to define an amount of liquid to be introduced. This implies that the amount of liquid that is introduced is carefully controlled.

The method is especially suited for extraction of bitter from beer. In such case, the first liquid is beer, the substance is bitter, and the second liquid is iso-octane.

According to another aspect of the invention, a device for liquid-liquid extraction comprises: a container, which is arranged to receive a first liquid comprising a substance to be extracted and a second liquid into which the substance is to be extracted. The second liquid is substantially immiscible with the first liquid and exhibits a preferential affinity to the substance to be extracted. The device further comprises a holder in which the container is mounted, and an actuating system. The actuating system is connected to the holder for accomplishing a reciprocating movement of the container, for keeping the holder at standstill to allow the liquids to settle thereby establishing an interface between the liquids, and for accomplishing a rotating movement around to move the interface over substantially the entire surface of the container.

The device provides a set-up for accomplishing a movement such that the liquids may be thoroughly mixed in the container and effectively separated after the extraction has been completed. By means of the device a reproducible mixing and separation of the liquids may be performed such that the liquid-liquid extraction is achieved in a controlled manner.

The device may further comprise a piston forming a bottom of the container, wherein the piston is moveable for increasing the volume of the container in order to allow liquid into the container filling the increased volume. Thus, the piston may be used to control the volume of liquid being introduced into the container.

The device may further comprise a step motor, which is connected to the piston for controlling the movement of the piston and accurately controlling volumes of liquid introduced into the container. This provides a very precise and accurate control of the volumes of liquids to be subjected to the liquid-liquid extraction.

Also, the piston may control output of liquid from the container in a precise manner. Using the step motor, a very accurate amount of liquid may be pushed out of the container by means of the piston.

The device may further comprise a flexible tubing, which is connected to a port of the container. This implies that the container may be subjected to vigorous movements while still being connected to a tubing for introducing liquids into the container.

The device may further comprise a selector valve, which is connected to the flexible tubing. This provides a possibility to easily introduce different liquids into the container via the flexible tubing.

The selector valve may thus be connected to liquid reservoirs for selectively providing introduction of liquids into the container.

The selector valve may comprise a plug for sealing the port of the container. Thus, the plug of the selector valve may be chosen during shaking and rotation, whereby it is ensured that no liquids are allowed to escape the container.

The selector valve may further be connected to an analysis instrument for providing output of the second liquid from the container to analysis after liquid-liquid extraction is completed. Thus, both input to and output from the container may be controlled by the selector valve.

### Brief Description of the Drawings

The invention will now by way of example be described in further detail with reference to the accompanying drawings.
Fig. 1 is a perspective view of a device according to an embodiment of the invention.
Fig. 2 is a flow chart of a method according to an embodiment of the invention.
Fig. 3 is another perspective view of the device of Fig. 1, wherein rotation of a container is illustrated.

### Detailed Description of a Presently Preferred Embodiment

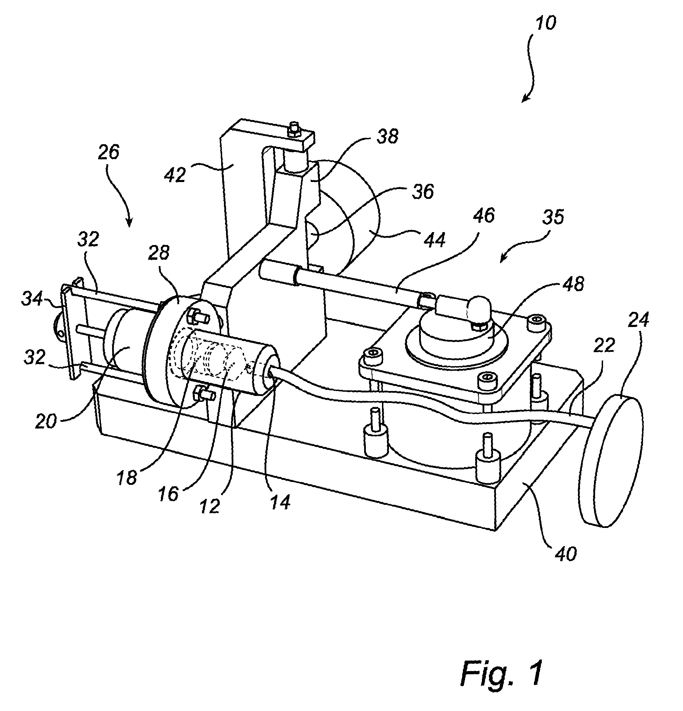

Referring now to Fig. 1, a device 10 for liquid-liquid extraction according to an embodiment of the invention will be described. The device 10 comprises a container 12, wherein liquid-liquid extraction is to be performed. The container 12 comprises an inlet/outlet port 14 and a cavity 16 in communication with the port 14. The cavity 16 is an elongate cylindrical space defined inside the container 12. A base of the cylindrical cavity 16 is formed by a piston 18 and the piston 18 is moveable for changing the volume of the cavity 16.

The port 14 of the container 12 may be narrow such that capillary action may draw liquid into the container 12. This implies that liquid may be easily introduced into the container. The port 14 is connected to a flexible tubing 22, which leads to a selector valve 24. The selector valve 24 provides selective connections to liquid reservoirs, a plug and an analysis instrument. The container 12 may thus selectively be connected to different liquid reservoirs for introducing liquids into the container 12. The container 12 may further be connected to an analysis instrument for outputting a liquid to be analysed. Further, the plug of the selector valve may provide that the container 12 is sealed such that no liquid escapes the container 12 during movement thereof.

The container 12 is mounted in a holder 26. The holder 26 comprises a ring 28 inside which the container 12 is mounted. The holder 26 thereby encloses the container 12 for secure mounting. The holder 26 further comprises two bars 32 which are fixedly attached to diametrically opposite positions of the ring 28. A plate 34 is slidably mounted on the bars 32. The plate 34 is also connected to the piston 18. The piston 18 is connected to a step motor 20 for controlling the position of the piston 18. Since the piston 18 is connected to the plate 34, the rotation of the piston 18 is prevented as the step motor 20 rotates to move the piston 18 in the cavity 16. Instead, a linear movement of the piston 18 is achieved. By means of the step motor 20 and the piston 18, the volume of the cavity 16 may be accurately controlled.

The device further comprises an actuating system 35 for controlling movements of the container. The holder 26 is connected to a shaft 36 of the actuating system 35. The shaft 36 is arranged in a support 38, which is rotably mounted to a base 40 of the device and to a bracket 42. When the support 38 rotates back and forth, it will induce a reciprocating movement of the holder 26 via the shaft 36.

The support 38 is also connected to an arm 46, which is connected to a motor 48 of the actuating system 35. The arm 46 is arranged off the rotational axis of the support 38, such that when the arm 46 is forced to move with the rotating motor 48, the support 38 will rotate back and forth and thus induce a reciprocating movement of the holder 26 and the container 12 in the holder 26.

The shaft 36 is further rotatably arranged in the support 38. This allows the shaft to be rotated for rotating the holder 26 around an axis forming a diameter of the ring 28. Another motor 44 of the actuating system 35 drives rotation of the shaft 36 in order to achieve rotation of the holder 26.

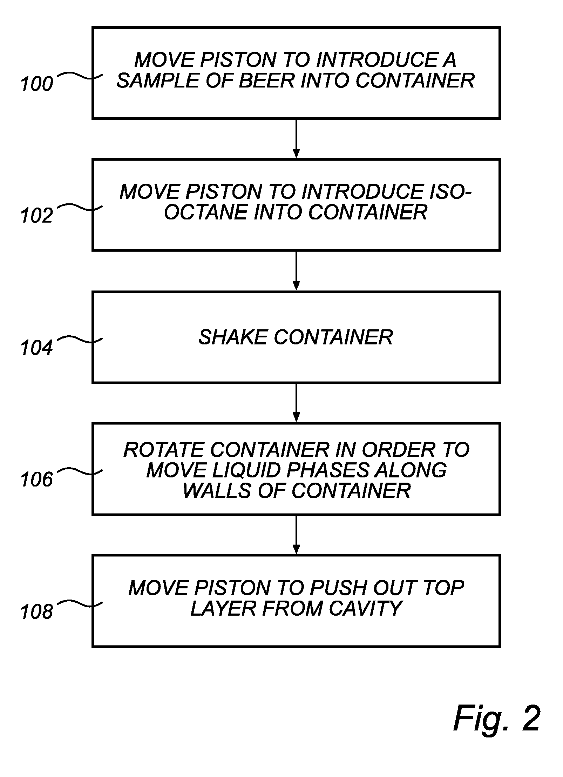

Referring now to Fig. 2, a method of liquid-liquid extraction will be described. The method is described with reference of extracting bitter from beer, but may be equally applied to other applications of liquid-liquid extraction.

Before extraction may be started, it is ensured that the container 12 is completely emptied from previous extractions. An overpressure of air is forced into the container 12 through the selector valve 24 and the flexible tubing 22. The container 12 is then rotated such that the port 14 is facing downwards. Thus, when the piston 18 is pushed to minimize the space of the cavity 16, any residual liquid will be forced out of the cavity 16.

Thereafter, the selector valve 24 is switched such that the container 12 is in connection with a beer reservoir. A sample of beer is introduced into the container 12, step 100, by moving the piston 18 to increase the volume of the cavity 16 such that an underpressure is created in the cavity 16. Thereafter, the selector valve 24 is switched such that the container 12 is in connection with another liquid reservoir. Then, the piston 18 is again moved to introduce hydrochloric acid into the container 12. The hydrochloric acid will act to break bonds between bitter and the beer. Once again, the selector valve 24 is swhitched such that the container 12 is in connection with yet another liquid reservoir. Again, the piston 18 is moved to introduce iso-octane into the container 12, step 102.

The selector valve 24 is now switched such that the container 12 is sealed. The container 12 is arranged in a position such that the longitudinal axis of the cavity 16 is horizontally arranged. The motor 48 is now activated such that a reciprocating movement of the container 12 is achieved. In this way, the container 12 is shaken, step 104, in order to mix the liquids in the container 12.

Iso-octane is immiscible in beer. The shaking action will increase the contact surface between iso-octane and beer. Further, the hydrochloric acid will act to break bonds between bitter and beer. Therefore, during shaking the bitter will be extracted from the beer into the iso-octane. Shaking is performed to allow all bitter to be extracted. A suitable time period for ascertaining that complete extraction is performed may be established by empirical studies.

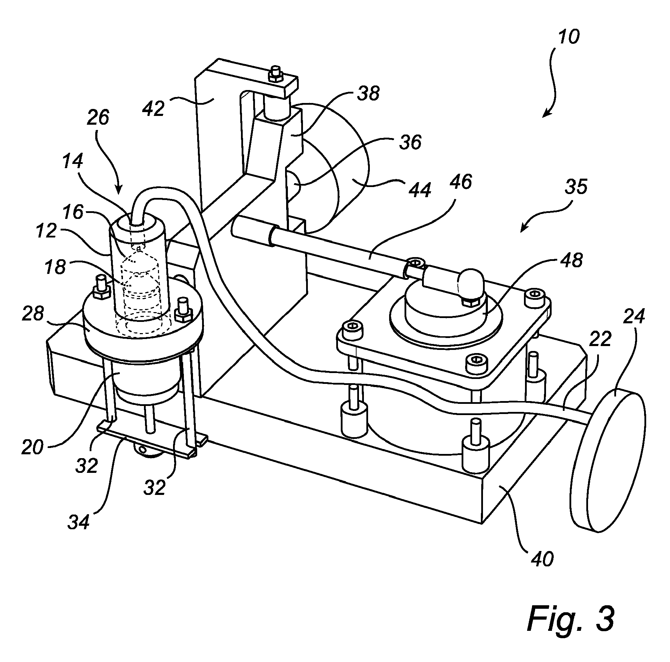

When the extraction is complete, the motor 48 is stopped. The iso-octane and the beer will separate as the liquids are allowed to settle, since the beer is heavier than iso-octane. Drops of iso-octane may however be trapped along the walls of the cavity 16 inside the beer phase. Thus, the shaft 36 is rotated 180° in order to move the liquid phases along the walls of the cavity 16, step 106. The shaft 36 is thereafter rotated back 90° such that the container 12 is arranged with the port 14 facing upwards, as illustrated in Fig. 3. Alternatively, the container 12 may be continuously rotated through 370°.

The iso-octane is now arranged at the top portion of the container 12 closest to the port 14. The selector valve 24 is switched so that the container 12 is connected with an analysis instrument. The iso-octane with the bitter may thus be outputted by moving the piston 18 to decrease the volume of the cavity 16, thus pushing out the top layer in the cavity 16, step 108. The iso-octane is thus transported into a measurement column in an analysis instrument. The concentration of bitter in the beer may thus be determined by measuring the absorbance of the outputted sample at a wavelength of 270 nm. The absorption may be related to the amount of bitter in the sample, since iso-octane is essentially transparent to the measurement wavelength. The liquid-liquid extraction has separated bitter from other compounds in the beer that would have distorted results of a direct absorption measurement on the beer.

It should be emphasized that the preferred embodiment described herein is in no way limiting and that many alternative embodiments are possible within the scope of protection defined by the appended claims.

## Claims

**1.** A method for liquid-liquid extraction, the method comprising:
introducing a first liquid into a container,
introducing a second liquid into the container being substantially immiscible with the first liquid and wherein a one of the first and second liquid contains a substance to be extracted and the other of the first and the second liquid exhibits a preferential affinity to the substance to be extracted,
shaking the container,
allowing the liquids to settle thereby establishing an interface between the liquids, and
rotating the container to move the interface over substantially the entire inner surface of the container.

**2.** The method according to claim 1, wherein said shaking is performed along a horizontal axis.

**3.** The method according to claim 1 or 2, wherein the container is rotated around a horizontal axis.

**4.** The method according to any one of the preceding claims, wherein the container is rotated at least through an angle of 180°.

**6.** The method according to any one of the preceding claims, wherein said rotating comprises rotating the container at least through an angle of 180° in a first direction and at least through an angle of 150° in a second, opposite direction.

**7.** The method according to any one of the preceding claims, wherein said rotating comprises stopping the container is arranged in an upright position and a port of the container is facing upwards.

**8.** The method according to any one of the preceding claims, further comprising introducing a third liquid into the container, which third liquid acts to break connections between the substance and the first liquid.

**9.** The method according to any one of the preceding claims, wherein said introducing of liquids is controlled by increasing a space in the container in order to define an amount of liquid to be introduced.

**10.** The method according to any one of the preceding claims, wherein said first liquid is beer, said substance is bitter, and said second liquid is iso-octane.

**11.** A device for liquid-liquid extraction, the device comprising:
a container, which is arranged to receive a first liquid comprising a substance to be extracted and a second liquid into which the substance is to be extracted, said second liquid being substantially immiscible with the first liquid and exhibiting a preferential affinity to the substance to be extracted,
a holder in which said container is mounted, and
an actuating system,
wherein said actuating system is connected to said holder for accomplishing a reciprocating movement of the container, for keeping the holder at standstill to allow the liquids to settle thereby establishing an interface between the liquids, and for accomplishing a rotating movement to move the interface over substantially the entire inner surface of the container.

**12.** The device according to claim 11, further comprising a piston forming a bottom of the container, wherein said piston is moveable for increasing the volume of the container in order to allow liquid into the container filling the increased volume.

**13.** The device according to claim 12, further comprising a step motor, which is connected to the piston for controlling the movement of the piston and accurately controlling volumes of liquid introduced into the container.

**14.** The device according to any one of claims 11-13, further comprising a flexible tubing, which is connected to a port of the container.

**15.** The device according to claim 14, further comprising a selector valve, which is connected to the flexible tubing.

**16.** The device according to claim 15, wherein the selector valve is connected to liquid reservoirs for selectively providing introduction of liquids into the container.

**17.** The device according to claim 15 or 16, wherein the selector valve comprises a plug for sealing the port of the container.

**18.** The device according to any one of claims 15-17, wherein the selector valve is connected to an analysis instrument for providing output of the second liquid from the container to analysis after liquid-liquid extraction is completed.
